(19) Europäisches Patentamt
European Patent Office
Office européen des brevets

(11) Veröffentlichungsnummer: **0 235 347 B1**

(12) **EUROPÄISCHE PATENTSCHRIFT**

(45) Veröffentlichungstag der Patentschrift: **09.10.91**

(51) Int. Cl.5: **C07D 491/04**, A61K 31/44, //(C07D491/04,307:00,211:00)

(21) Anmeldenummer: **86110896.7**

(22) Anmeldetag: **06.08.86**

(54) **Dihydropyridinlactole, Verfahren zur Herstellung und ihre Verwendung in Arzneimitteln.**

(30) Priorität: **17.01.86 DE 3601226**

(43) Veröffentlichungstag der Anmeldung: **09.09.87 Patentblatt 87/37**

(45) Bekanntmachung des Hinweises auf die Patenterteilung: **09.10.91 Patentblatt 91/41**

(84) Benannte Vertragsstaaten: **AT BE CH DE FR GB IT LI LU NL SE**

(56) Entgegenhaltungen:
**EP-A- 0 080 220**
**EP-A- 0 111 455**
**EP-A- 0 144 847**

(73) Patentinhaber: **BAYER AG**

**W-5090 Leverkusen 1 Bayerwerk(DE)**

(72) Erfinder: **Goldmann, Siegfried, Dr.**
**Am Osterholz 91**
**W-5600 Wuppertal 11(DE)**
Erfinder: **Bossert, Friedrich, Dr.**
**Claudiusweg 7**
**W-5600 Wuppertal 1(DE)**
Erfinder: **Ahr, Hans Jürgen, Dr. Dr.**
**Claudiusweg 3**
**W-5600 Wuppertal 1(DE)**
Erfinder: **Bischoff, Hilmar, Dr.**
**Wilkhausstrasse 107**
**W-5600 Wuppertal 2(DE)**
Erfinder: **Puls, Walter, Dr.**
**In den Birken 75**
**W-5600 Wuppertal 1(DE)**
Erfinder: **Petzinna, Dieter, Dr. Dr.**
**Baustrasse 26**
**W-4230 Wesel(DE)**
Erfinder: **Schlossmann, Klaus, Dr.**
**In der Beek 116**
**W-5600 Wuppertal 1(DE)**
Erfinder: **Bender, Joachim, Dr.**
**Steinberger Weg 52**
**W-5600 Wuppertal 11(DE)**

**EP 0 235 347 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Dihydropyridinlactole, Verfahren zu ihrer Herstellung sowie ihre Verwendung in Arzneimitteln, insbesondere von blutzucker- und kreislaufbeeinflussenden Arzneimitteln.

Es sind bereits Dihydropyridinlactone bekannt aus EP-A 229 964, EP-A 144 847 und EP-A 111 455, für die auch eine Blutzuckerwirkung beschrieben ist, die sich aber durch das Fehlen der Hydroxygruppe in 7-Position strukturell von den erlindungsgemäßen Verbindungen unterscheiden. Gleichzeitig ist für Dihydropyridine bekannt, daß geringfügige Strukturmodifikationen gravierenden Wirkungsänderungen zur Folge haben können. EP-A 80 220 beschreibt z. B. Dihydropyridine, die in 7-Stellung anders substituiert sind und eine gegenteilige Wirkung aufweisen. Es konnte daher nicht erwartet werden, daß durch Einführung der Hydroxygruppe in 7-Position eine solche vorteilhafte Wirkung erreicht wird.

Die vorliegende Erfindung betrifft Dihydropyridinlactole der allgemeinen Formel I,

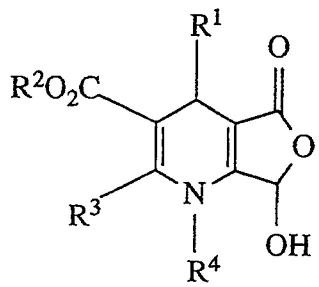

(I)

in welcher

$R^1$ für einen Phenylrest steht, der gegebenenfalls substituiert ist durch Nitro, Chlor, Trifluor-$C_1$-$C_3$alkyl, $C_1$-$C_3$-Alkyl,

$R^2$ für einen $C_1$-$C_4$-Alkylrest steht, der gegebenenfalls substituiert ist durch $C_1$-$C_2$-Alkoxy,

$R^3$ für eine $C_1$-$C_3$-Alkylgruppe steht, und

$R^4$ für eine $C_1$-$C_4$-Alkylgruppe steht,

in Form ihrer Isomeren, Isomerengemische, optischen Antipoden oder Racemate sowie ihre physiologisch unbedenklichen Salze.

Die erfindungsgemäßen Verbindungen können in Form ihrer Salze vorliegen. Im allgemeinen sind dies Salze der erfindungsgemäßen Stoffe mit anorganischen oder organischen Säuren. Bevorzugt sind jedoch die physiologisch unbedenklichen Salze der erfindungsgemäßen Stoffe mit anorganischen oder organischen Säuren. Als Beispiele seien genannt : Hydrohalogenide, Hydrogensulfate, Sulfate, Hydrogenphosphate, Acetate, Maleate, Citrate, Fumarate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie Diastereomerengemische. Die Racemformen lassen sich ebenso wie die Diastereomeren in bekannter Weise in die stereoisomer einheitliche Bestandteile trennen (vgl. E.L. Eliel, Stereochemistry of Carbon Compounds McGraw Hill, 1962).

Die erfindungdsgemäßen Verbindungen der allgemeinen Formel I werden hergestellt,
in dem man

[A] Formylverbindungen der allgemeinen Formel (II),

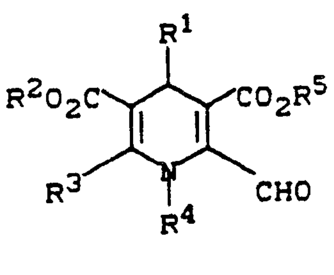

(II)

in welcher

$R^1$-$R^4$ - die angegebene Bedeutung haben und

$R^5$ - für geradkettiges oder verzweigtes Alkyl mit bis zu 8 Kohlenstoffatomen steht,

in geeigneten Lösungsmitteln zunächst mit Base und dann mit Säure umsetzt,
oder indem man
[B] Dihydropyridinlactone der allgemeinen Formel (III),

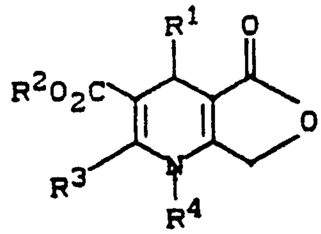

(III)

in welcher
$R^1$-$R^4$ - die angegebene Bedeutung haben,
in geeigneten Lösungsmitteln gegebenenfalls in Anwesenheit einer Base bromiert und anschließend hydrolysiert oder direkt hydroxyliert.

Je nach Art der verwendeten Ausgangsstoffe kann die Herstellung der erfindungsgemäßen Verbindungen nach Verfahren A und B durch folgende Schemata verdeutlicht werden:

[A]

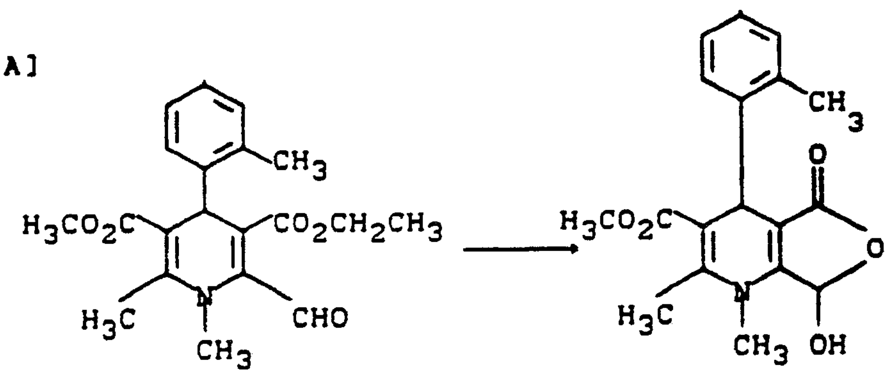

[B]

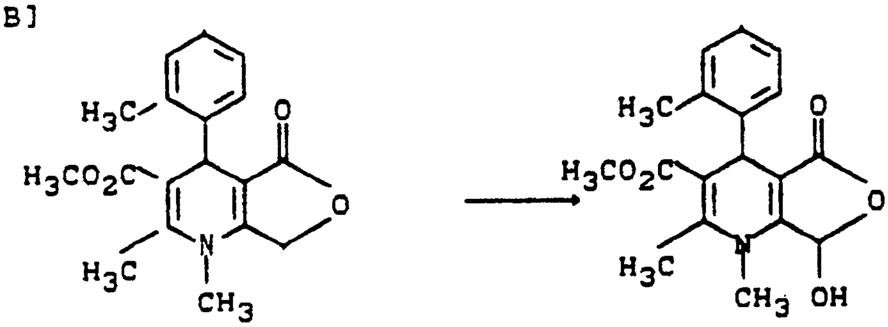

<u>Verfahren A</u>

Als Lösungsmittel für Verfahren A eignen sich Wasser sowie alle inerten organischen Lösungsmittel, die sich unter den Reaktionsbedingungen nicht verändern. Hierzu gehören bevorzugt Alkohole wie Methanol, Ethanol, Propanol oder Isopropanol, Ether wie Diethylether, Dioxan, Tetrahydrofuran, Glykolmono- oder dimethylether, Acetonitril, Pyridin, Dimethylformamid, Dimethylsulfoxid oder Hexamethylphosphorsäuretriamid. Ebenso ist es möglich Gemische der genannten Lösungsmittel zu verwenden.

Als Basen eignen sich bei Verfahren A die üblichen anorganischen oder organischen Basen. Hierzu gehören bevorzugt Alkalihydroxide wie Natriumhydroxid oder Kaliumhydroxid, oder Alkalialkoholate wie Natriummethylat, Natriumethylat, Kaliumethylat, Kaliummethylat oder Kalium-tert.-butanolat, Alkalimetalle wie Natrium, Alkalihydride wie Natriumhydrid oder Kaliumhydrid, Alkaliamide wie Natriumamid oder Lithiumdiisopropylamid.

Als Säuren kommen die üblichen organischen oder anorganischen Säuren in Frage. Hierzu gehören bevorzugt Mineralsäuren wie Chlorwasserstoffsäure, Bromwasserstoffsäure, Schwefelsäure, Phosphorsäure oder organische Carbonsäuren wie Essigsäure.

Die Durchführung des Verfahrens A erfolgt, indem man zunächst die Formylverbindungen der Formel (II) in geeigneten Lösungsmitteln mit 100 bis 5 Mol, bevorzugt von 50 bis 10 mol Base bezogen auf 1 Mol Formylverbindung umsetzt und anschließend das Reaktionsgemisch mit Säuren behandelt. Die Aufarbeitung erfolgt in üblicher, dem Fachmann vertrauter Weise.

Die Umsetzung wird im allgemeinen bei Temperaturen von 0° C bis 150° C, bevorzugt von 20° C bis 100° C durchgeführt.

Die Umsetzung kann bei normalem, aber auch bei erhöhtem oder erniedrigtem Druck erfolgen. Im allgemeinen arbeitet man bei normalem Druck.

Die Ausgangsverbindungender Formel (II) sind bekannt oder können nach bekannten Verfahren hergestellt werden (vgl. DOS 26 29 892).

Verfahren B

Die Bromierung erfolgt in an sich bekannter Weise mit den üblichen Bromierungsmitteln wie N-Bromsuccinimid oder Brom, vorzugsweise mit Brom.

Als Base eignen sich hierbei die üblichen Basen. Hierzu gehören bevorzugt Alkalimetalle wie Natrium oder Kalium, Alkalimetallhydride wie Natriumhydrid oder Kaliumhydrid, Alkaliamide wie Natriumamid oder Lithiumdiisopropylamid oder metallorganische Verbindungen wie Phenyllithium, n-Butyllithium oder tert.-Butyllithium, Alkoholate wie Na-ethanolat oder Kalium-t-butylat.

Als Lösungsmittel eignen sich hierbei die üblichen inerten organischen Lösungsmittel. Hierzu gehören bevorzugt Ether wie Diethylether, Dioxan oder Tetrahydrofuran oder Kohlenwasserstoffe wie Benzol, Toluol, Xylol, Hexan, Cyclohexan oder Erdölfraktionen. Es ist ebenso möglich, Gemische der genannten Lösungsmittel einzusetzen.

Die Bromierung wird in einem Temperaturbereich von -120° C bis 100° C, bevorzugt von -78° C bis 50° C durchgeführt.

Die Bromierung erfolgt derart, daß man zunächst mit 5 bis 1 mol, bevorzugt mit 2 bis 1 mol, besonders bevorzugt mit 1 mol Base bezogen auf 1 mol der Ausgangsverbindung (III) ein Anion erzeugt und dieses mittels Brom in Bromid überführt. Die anschließende Überführung der Bromverbindung zur entsprechenden Hydroxyverbindung der Formel (I) wird zweckmäßigerweise ohne Isolierung der Bromverbindung durchgeführt. Diese Hydrolyse erfolgt in an sich bekannter Weise durch Wasser, gegebenenfalls in Anwesenheit von Spuren einer Säure wie Salzsäure oder Schwefelsäure.

Das Verfahren B kann sowohl bei normalem als auch bei erhöhtem oder erniedrigtem Druck durchgeführt werden. Im allgemeinen arbeitet man bei Normaldruck.

Die Überführung der Verbindungen (III) in die erfindungsgemäßen Verbindungen (I), kann jedoch auch nach anderen literaturbekannten Methoden durchgeführt werden und ist nicht auf die angegebenen Verfahren beschränkt.

Die Hydroxylierung kann ebenso durch 2-Sulfonyloxaziridin, mit Molybdänperoxid/Pyridin/Phosphit oder mit Sauerstoff/Phosphit jeweils in Anwesenheit von Basen, in inerten organischen Lösungsmitteln durchgeführt werden, wie es beispielsweise durch E.Vedejs in J.Am.Chem. Soc. 96, 5944 (1974) oder J. Org. Chem. 43, 188 (1978), oder durch J.M. Billmers, J. Finn in J. Org. Chem. 49, 3243 (1984) oder durch H.H. Wassermann, B.H. Lipschutz in Tetrahedron Letters 1975, 1731 beschrieben wird.

Die erfindungsgemäßen Verbindungen der Formel I zeigen ein wertvolles pharmakologisches Wirkspektrum.

Diese Verbindungen senken den Blutzucker und können so zur Behandlung von Diabetes eingesetzt werden.

Die blutglucosesenkende Wirkung der zu untersuchenden Substanzen wurde an männlichen Wistar-Ratten mit einem Gewicht zwischen 140 und 190 g geprüft. Die Ratten wurden zu diesem Zweck 18 h vor der Applikation der Substanzen gewogen, in Gruppen zu 6 Tieren eingeteilt und nüchtern gesetzt. Die zu untersuchenden Substanzen wurden direkt vor der Applikation in wäßriger 0,75 %iger Traganthsuspension mit einem Ultra-Turrax suspendiert. Die Applikation der Traganthsuspension (Kontrolltiere) bzw. der in Traganth suspendierten Substanzen erfolgte mittels einer Schlundsonde.

Die Blutentnahme erfolgt bei jeder Ratte 30, 60 und 120 min nach der Applikation aus dem retroorbitalen Venenplexus. Jeweils 30 µl Blut wurden mit einem automatischen Dilutor entnommen und mit 0,3 ml Uranylacetat (0,16%) enteiweißt. Nach der Zentrifugation wurden die Glucose im Überstand nach der

Glucoseoxidase-Methode mit 4-Amino-phenazon als Farbreagenz an einem Gemsaec Fastanalyzer photometrisch bestimmt. Die Auswertung der Ergebnisse erfolgt mit dem t-Test nach Student, als Signifikanzgrenze wurde $p < 0,05$ gewählt.

Substanzen, die bei Ratten zu einem Zeitpunkt eine signifikante Reduktion der Blutglucosekonzentration um mindestens 10 % bewirkten, verglichen mit der Kontrollgruppe, die nur Traganthsuspension erhielt, wurden als wirksam angegeben.

Die nachfolgende Tabelle 1 enthält die gefundenen Veränderungen der Blutglucosekonzentrationen in Prozent der Kontrolle.

Tabelle 1

| Substanz (Patentbeispiel Nr.) | Abnahme der Blutglucosekonzentration in % der Kontrolle 10 mg/kg p.o. |
|---|---|
| 1 | 29 |
| 2 | 34 |

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einen Bruchteil oder einem Vielfachen einer Einzeldosis entspricht. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen genannt.

Tabletten, Dragees, Kapseln, Pillen und Granulate können den oder die Wirkstoffe neben den üblichen Trägerstoffen enthalten, wie (a) Füll- und Streckmittel, z.B. Stärke, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole, oder Gemische der unter (a) bis (i) aufgeführten Stoffe.

Die Tabletten, Dragees, Kapseln, Pillen und Granulate können mit den üblichen gegebenenfalls Opakisierungsmitteln enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen, z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-

Alkohol mit $C_{16}$-Fettsäure) oder Gemische dieser Stoffe.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Traganten oder Gemische dieser Stoffe enthalten.

Die genannten Formulierungsformen können auch Färbemittel, Konservierungsmittel sowie geruchs- und geschmachsverbessernde Zusätze, z.B. Pfefferminzöl und Eukalyptusöl und Süßmittel, z.B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der Verbindungen der Formel I und/oder deren Salze sowie von pharmazeutischen Zubereitungen, die die Verbindungen der Formel I und/oder deren Salze enthalten, in der Human- und Veterinärmedizin zur Verhütung, Verbesserung und/oder Heilung der oben aufgeführten Erkrankungen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,01 bis etwa 200 mg/kg, vorzugsweise 0,1 bis 50 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den vorgenannten Dosierungen abzuweichen und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

## Herstellungsbeispiele

### Beispiel 1 (Verfahren B)

4-(2-Chlorphenyl)-1-ethyl-7-hydroxy-2-methyl-5-oxo-1.4.5.7-tetrahydrofuro[2,4-b]pyridin-3-carbonsäureisopropylester

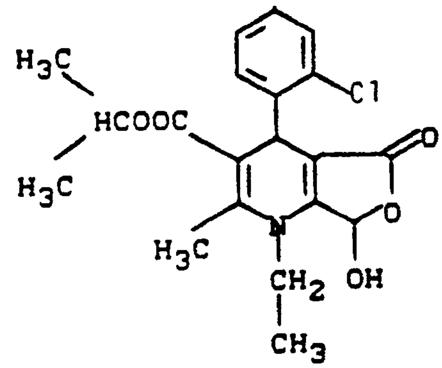

60 mmol Diisopropylamin werden in 100 ml Tetrahydrofuran p.a. vorgelegt. Bei einer Temperatur von 0 °C werden unter $N_2$-Strom 50 mmol Butyl lithium zugegeben. Danach wird auf -78 °C gekühlt und eine Lösung aus 50 mmol 4-(2-Chlorphenyl)-1-ethyl-2-methyl-5-oxo-1.4.5.7-tetrahydrofuro[3.4-b]pyridin-3-carbonsäureisopropylester (in THF gelöst) zugetropft. Es wird 15 min bei -78 °C gerührt und mit Hilfe von Stickstoff wird diese Lösung in eine Lösung von 50 mmol $Br_2$ und 50 ml THF gepumpt, sofort danach gibt man 50 mmol Cyclohexen dazu und läßt auf Raumtemperatur erwärmt, engt ein, löst in DMSO und gibt bis zur beginnenden Trübung $H_2O$ hinzu. Man läßt 12 Stunden stehen, fällt mit $H_2O$ aus, saugt ab und trennt mit $CHCl_3$/MeOH 9:1 an Kieselgel.
Ausbeute: 30 % der Theorie
Fp.: 145-147 °C.

Analog Beispiel 1 wurden hergestellt:

Beispiel 2

4-(2-Chlorphenyl)-1-ethyl-7-hydroxy-2-methyl-5-oxo-1.4.5.7-tetrahydrofuro[3.4-b]pyridin-3-carbonsäureethylester

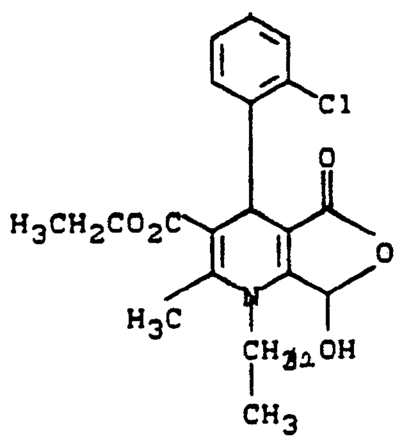

Ausbeute: 13 % der Theorie (amorph)
MS: 377 (10 %, $M^+$); 348 (20 %); 304 (10 %); 266 (100 %); 29 (40 %)

Beispiel 3

1-Ethyl-7-hydroxy-2-methyl-4-(2-methylphenyl)-5-oxo-1,4,5,7-tetrahydrofuro[3,4-b]pyridin-3-carbonsäure-isopropylester

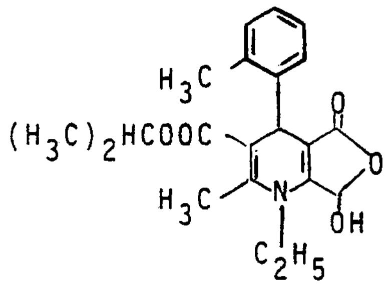

Ausbeute: 30 % der Theorie
MS: 371 (15 %, $M^+$); 328 (25 %); 280 (80 %); 278 (100 %); 192 (20 %); 164 (20 %); 42 (50 %); 29 (35 %)

Beispiel 4

1-Ethyl-7-hydroxy-2-methyl-5-oxo-4-(2-Trifluormethylphenyl) -1,4,5,7-tetrahydrofuro[3,4-b]-pyridin-3-carbonsäureisopropylester

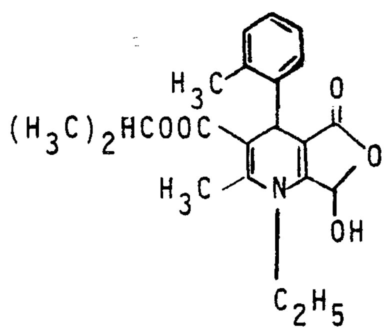

Ausbeute: 35 % der Theorie

1H-NMR ($CDCl_3$) : δ = 0,8 (d, 3H); 1,1 (d, 3H); 1,3 (t, 3H); 2,3 (s, 3H); 3,5-3,9 (s, breit, 2H); 4,8 (hept., 1H); 5,3 (s, 1H), 5,9 (s,1H); 6,1 (s, breit, 1H); 7,2-7,6 (m, 4H);

Beispiel 5 (Verfahren A)

1-Ethyl-7-hydroxy-2-methyl-4-(3-nitrophenyl)-5-oxo-1,4,5,7-tetrahydrofuro[3,4-b]-pyridin-3-carbonsäureisopropylester

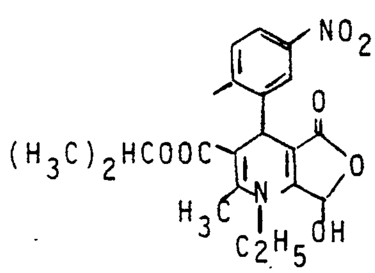

5 mmol 1-Ethyl-2-formyl-6-methyl-4-(3-nitrophenyl)-1,4-dihydropyridin-3,5-dicarbonsäure-3-methylester-5-isopropylester werden in 40 mmol 2 N KOH vorgelegt und im Wasserbad kurz auf 50 °C erwärmt, danach läßt man 1 h bei Raumtemperatur nachrühren. Die Lösung wird mit Aktivkohle geklärt und mit Salzsäure angesäuert, der Niederschlag wird abgesaugt.

Ausbeute: 25 % der Theorie
1H-NMR ($CDCl_3$) : δ = 0,8 (d, 3H); 1,1 (d, 3H); 1,3 (t, 3H); 2,3 (s, 3H); 3,4-4,0 (m, breit, 2H); 4,7 (m,

1H); 5,1 (s,1H); 5,9 (s, 1H); 6,2 (s, breit, 1H(; 7,3-8,3 (m, 4H);

**Patentansprüche**

1. Dihydropyridinlactole gemäß der allgemeinen Formel I,

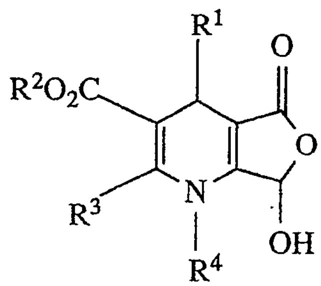

(I)

in welcher
$R^1$ für einen Phenylrest steht, der gegebenenfalls substituiert ist durch Nitro, Chlor, Trifluor-$C_1$-$C_3$-alkyl, $C_1$-$C_3$-Alkyl,
$R^2$ für einen $C_1$-$C_4$-Alkylrest steht, der gegebenenfalls substituiert ist durch $C_1$-$C_2$-Alkoxy,
$R^3$ für eine $C_1$-$C_3$-Alkylgruppe steht, und
$R^4$ für eine $C_1$-$C_4$-Alkylgruppe steht,
in Form ihrer Isomeren, Isomerengemische, optischen Antipoden oder Racemate sowie ihre physiologisch unbedenklichen Salze.

2. Dihydropyridinlactole gemäß Anspruch 1 zur Bekämpfung von Erkrankungen.

3. Arzneimittel enthaltend als Wirkstoff ein Dihydropyridinlactol gemäß Anspruch 1.

4. Verfahren zur Herstellung eines Dihydropyridinlactols der allgemeinen Formel I

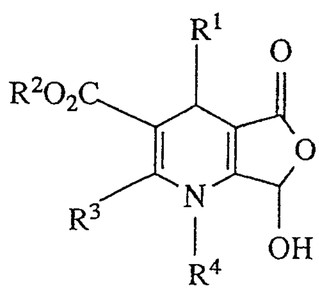

(I)

in welcher
$R^1$ für einen Phenylrest steht, der gegebenenfalls substituiert ist durch Nitro, Chlor, Trifluor-$C_1$-$C_3$-alkyl, $C_1$-$C_3$-Alkyl,
$R^2$ für einen $C_1$-$C_4$-Alkylrest steht, der gegebenenfalls substituiert ist durch $C_1$-$C_2$-Alkoxy,
$R^3$ für eine $C_1$-$C_3$-Alkylgruppe steht, und
$R^4$ für eine $C_1$-$C_4$-Alkylgruppe steht,
in Form ihrer Isomeren, Isomerengemische, optischen Antipoden oder Racemate sowie ihre physiologisch unbedenklichen Salze, dadurch gekennzeichnet, daß man

[A] Formylverbindungen der allgemeinen Formel (II)

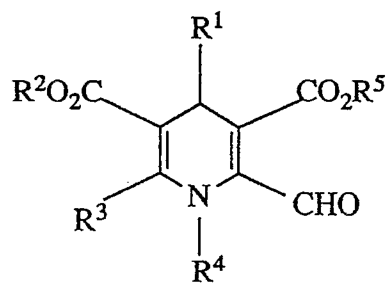

(II)

in welcher

$R^1$-$R^4$ die angegebene Bedeutung haben und

$R^5$ für geradkettiges oder verzweigtes Alkyl mit bis zu 8 Kohlenstoffatomen steht,

in geeigneten Lösungsmitteln zunächstmit Base und dann mit Säure umsetzt, oder indem man

[B] Dihydropyridinvlactone der allgemeinen Foreml III,

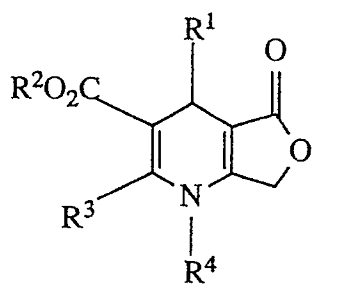

(III)

in welcher $R^1$-$R^4$ die angegebene Bedeutung haben,

in geeigneten Lösungsmitteln gegebenenfalls in Anwesenheit einer Base bromiert und anschließend hydrolysiert oder direkt hydroxyliert.

5. Verfahren zur Herstellung eines Arzneimittels gemäß Anspruch 3, dadurch gekennzeichnet, daß man den Wirkstoff mit üblichen Hilfs- und/oder Trägerstoffen mischt.

6. Verwendung von Dihydropyridinlactolen der allgemeinen Formel I gemäß Anspruch 1 zur Herstellung von Arzneimitteln mit blutzuckerbeeinflussender Wirkung.

## Claims

1. Dihydropyridinelactols according to the general formula 1,

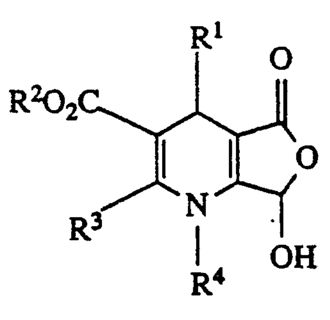

(I)

in which

$R^1$ represents a phenyl radical which is optionally substituted by nitro, chlorine, trifluoro-$C_1$-$C_3$-

alkylor $C_1$-$C_3$-alkyl,
$R^2$ represents a $C_1$-$C_4$-alkyl radical which is optionally substituted by $C_1$-$C_2$-alkoxy,
$R^3$ represents a $C_1$-$C_3$-alkyl group, and
$R^4$ represents a $C_1$-$C_4$-alkyl group,

in the form of their isomers, isomer mixtures, optical antipodes or racemates, and their physiologically acceptable salts.

**2.** Dihydropyridinelactols according to Claim 1 for combating diseases.

**3.** Medicaments containing, as an active compound, a dihydropyridinelactol according to Claim 1.

**4.** Process for the preparation of a dihydropyridinelactol of the general formula I

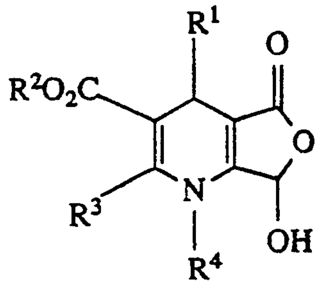

(I)

in which
$R^1$ represents a phenyl radical which is optionally substituted by nitro, chlorine, trifluoro-$C_1$-$C_3$-alkylor $C_1$-$C_3$-alkyl,
$R^2$ represents a $C_1$-$C_4$-alkyl radical which is optionally substituted by $C_1$-$C_2$-alkoxy,
$R^3$ represents a $C_1$-$C_3$-alkyl group, and
$R^4$ represents a $C_1$-$C_4$-alkyl group,

in the form of their isomers, isomer mixtures, optical antipodes or racemates, and their physiologically acceptable salts, characterised in that

[A] formyl compounds of the general formula (II)

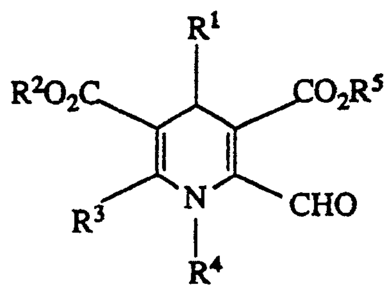

(II)

in which
$R^1$-$R^4$ have the stated meaning and
$R^5$ represents straight-chain or branched alkyl having up to 8 carbon atoms,

in suitable solvents, are first reacted with a base and then with an acid,

or

[B] dihydropyridinelactones of the general formula III,

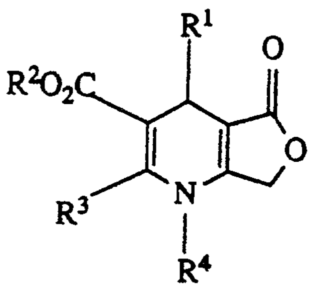

(III)

in which $R^1$-$R^4$ have the stated meaning,

are brominated in suitable solvents, if appropriate in the presence of a base, and then hydrolysed or directly hydroxylated.

5. Process for the preparation of a medicament according to Claim 3, characterised in that the active compound is mixed with customary auxiliaries and/or excipients.

6. Use of dihydropyridinelactols of the general formula I according to Claim 1 for the preparation of medicaments having an action which influences blood sugar.

**Revendications**

1. Dihydropyridinelactols selon la formule générale (I) :

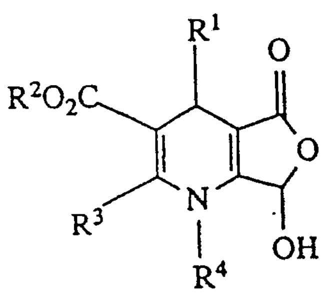

(I)

dans laquelle

$R^1$ représente un reste phényle qui est éventuellement substitué par un groupe nitro, un atome de chlore, un groupe trifluoroalkyle en $C_1$-$C_3$, alkyle en $C_1$-$C_3$,

$R^2$ représente un reste alkyle en $C_1$-$C_4$ qui est éventuellement substitué par un alcoxy en $C_1$-$C_2$,

$R^3$ représente un groupe alkyle en $C_1$-$C_3$ et

$R^4$ représente un groupe alkyle en $C_1$-$C_4$,

sous forme de leurs isomères, de leurs mélanges d'isomères, de leurs antipodes optiques ou de leur racémates ainsi que leurs sels irréprochables du point de vue physiologique.

2. Dihydropyridinelactols selon la revendication 1, pour combattre les maladies.

3. Médicament contenant comme substance active un dihydropyridinelactol selon la revendication 1.

4. Procédé de préparation d'un dihydropyridinelactol de formule générale (I) :

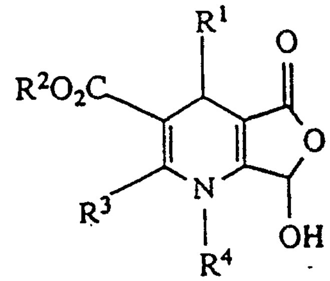

(I)

dans laquelle

$R^1$ représente un reste phényle qui est éventuellement substitué par un groupe nitro, un atome de chlore, un groupe trifluoroalkyle en $C_1$-$C_3$, alkyle en $C_1$-$C_3$,

$R^2$ représente un reste alkyle en $C_1$-$C_4$ qui est éventuellement substitué par un alcoxy en $C_1$-$C_2$,

$R^3$ représente un groupe alkyle en $C_1$-$C_3$, et

$R^4$ représente un groupe alkyle en $C_1$-$C_4$,

sous forme de ses isomères, de ses mélanges d'isomères, de ses antipodes optiques ou de ses racémates ainsi que ses sels irréprochables du point de vue physiologique, caractérisé en ce que

[A] l'on fait réagir des composés formylés de formule générale (II)

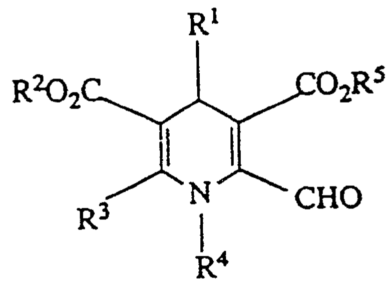

(II)

dans laquelle

$R^1$-$R^4$ ont la signification indiquée et

$R^5$ représente un alkyle linéaire ou ramifié contenant jusqu'à 8 atomes de carbone,

dans des solvants appropriés, d'abord avec une base puis avec un acide, ou en ce que

[B] l'on brome une dihydropyridinelactone de formule générale (III)

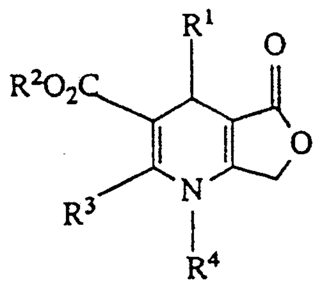

(III)

dans laquelle

$R^1$-$R^4$ ont la signification indiquée,

dans des solvants appropriés, éventuellement en présence d'une base, puis on l'hydrolyse ou on l'hydroxyle directement.

**5.** Procédé de préparation d'un médicament selon la revendication 3, caractérisé en ce que l'on mélange la substance active avec des adjuvants et/ou des véhicules habituels.

**6.** Utilisation des dihydropyridinelactols de formule générale (I) selon la revendication 1 pour la préparation de médicaments ayant un effet sur la glycémie.